# FASCICULE DE BREVET EUROPEEN

(11) **EP 3 738 596 B1**
(45) Date de publication et mention de la délivrance du brevet: **16.03.2022**
(21) Numéro de dépôt: 19174909.2
(22) Date de dépôt: 16.05.2019
(51) Int. Cl.: A61K 33/08, A61K 31/131, A61K 31/16, A61P 25/00

(54) **COMPOSÉ ET COMPOSITION POUR UTILISATION DANS LE TRAITEMENT DU SYNDROME PRÉMENSTRUEL ET/OU DU TROUBLE DYSPHORIQUE PRÉMENSTRUEL**
SUBSTANZ UND ZUSAMMENSETZUNG FÜR DEN EINSATZ ZUR BEHANDLUNG DES PRÄMENSTRUELLEN SYNDROMS UND/ODER DER PRÄMENSTRUELLEN DYSPHORISCHEN STÖRUNG
COMPOUND AND COMPOSITION FOR USE IN THE TREATMENT OF PREMENSTRUAL SYNDROME AND/OR PREMENSTRUAL DYSPHORIC DISORDER

(43) Date de publication de la demande: 18.11.2020
(73) Titulaire: Synapharm Industrial Synthesis, 4432 Alleur (BE)
(72) Inventeur: Danhier, Philippe, 7382 Audregnies (BE); Azzam, Pascale, 4000 Liège (BE)
(74) Mandataire: ABYOO

(56) Documents cités:
- EP-A1- 1 661 575
- WO-A1-2016/205089
- KR-A- 20160 008 257
- F FACCHINETTI: "Oral magnesium successfully relieves premenstrual mood changes", OBSTET GYNECOL., vol. 78, no. 2, 1 août 1991 (1991-08-01), pages 177-81, XP055642344,
- QUARANTA S ET AL: "Pilot study of the efficacy and safety of a modified-release magnesium 250mgtablet (Sincromag((R))) for the treatment of premenstrual syndrome", CLINICAL DRUG INVESTIGATION, ADIS INTERNATIONAL, AUCKLAND, NZ, vol. 27, no. 1, 1 janvier 2007 (2007-01-01), pages 51-58, XP009092161, ISSN: 1173-2563, DOI: 10.2165/00044011-200727010-00004
- UYSAL NAZAN ET AL: "Timeline (Bioavailability) of Magnesium Compounds in Hours: Which Magnesium Compound Works Best?", BIOLOGICAL TRACE ELEMENT RESEARCH, HUMANA PRESS, CLIFTON, NJ, US, vol. 187, no. 1, 21 avril 2018 (2018-04-21), pages 128-136, XP036665312, ISSN: 0163-4984, DOI: 10.1007/S12011-018-1351-9 Extrait de l'Internet: URL:https://link.springer.com/content/pdf/ 10.1007%2Fs12011-018-1351-9.pdf> [extrait le 2018-04-21]
- MARIA KHARITONOVA ET AL: "Comparative angioprotective effects of magnesium compounds", JOURNAL OF TRACE ELEMENTS IN MEDICINE AND BIOLOGY, vol. 29, 1 janvier 2015 (2015-01-01), pages 227-234, XP055642261, US ISSN: 0946-672X, DOI: 10.1016/j.jtemb.2014.06.026
- J Durlach: "Mg Acetyltaurinate as a photic inhibitor in photosensitive magnesium depletion: a physiological pathway in headache with photophobia treatment", , 1 janvier 2013 (2013-01-01), XP055485521, Extrait de l'Internet: URL:https://pdfs.semanticscholar.org/bd78/ 25ebb71f41bf84e60f84e5af9c65e03146c8.pdf?_ ga=2.77921361.439198391.1529396376-1410810 065.1502265378 [extrait le 2018-06-19]

## Description

La présente invention se rapporte à un composé et à une composition pour utilisation dans le traitement du syndrome prémenstruel et/ou du trouble dysphorique prémenstruel.

Le syndrome prémenstruel (ou SPM) est un ensemble de troubles survenant durant les jours qui précèdent ou pendant les menstruations. Il est caractérisé par des symptômes divers comme un gonflement douloureux des seins, des maux de tête, les jambes lourdes ou une prise de poids, des éruptions cutanées ou d'herpès et par des troubles du comportement ou troubles de l'humeur incluant nervosité, anxiété, agressivité, émotivité, dépression. Le trouble dysphorique prémenstruel (TDPM) est quant à lui reconnu comme étant une forme plus sévère du syndrome prémenstruel.

Une revue générale de la littérature (Zaafrane et al., Journal de Gynécologie Obstétrique et Biologie de la Reproduction, 36, 2007, 642-652) définit le syndrome prémenstruel comme étant un ensemble de symptômes somatiques, affectifs et comportementaux apparaissant avant les règles et disparaissant avec ces dernières. Cette revue précise que cet ensemble symptomatique toucherait approximativement 75% des femmes en âge de procréer et que, actuellement, les traitements de la symptomatologie prémenstruelle sont variés, d'indications peu nettes et d'efficacité peu établie.

Selon la même revue générale de la littérature, le syndrome prémenstruel peut associer plus d'une centaine de symptômes d'ordre somatique, cognitif, comportemental ou affectif. Toutefois, les plaintes les plus fréquentes se présentent sous forme d'une triade caractéristique :
a) tension mammaire : les seins sont tendus, sensibles, hypervascularisés, douloureux, pouvant gêner les mouvements du bras ;
b) tension abdominopelvienne : ballonnement abdominal plus ou moins prononcé, toujours gênant, souvent associé à une constipation, ou lourdeur pelvienne avec un inconfort du port des vêtements et prise de poids occasionnelle prémenstruelle ;
c) tension psychique : les troubles neuropsychiques les plus fréquents sont l'irritabilité, la labilité de l'humeur, l'humeur dépressive, l'anxiété, l'asthénie, des troubles du sommeil et du comportement alimentaire et des céphalées.

Le but du traitement dans le SPM est une réduction des symptômes et une amélioration de l'état général. Des traitements médicamenteux existent et reposent essentiellement sur une prise d'antidépresseurs et/ou d'anxiolytiques et/ou de contraceptifs et/ou de progestérone et/ou d'agonistes GnRH. Parmi les molécules utilisées se retrouvent les triptans (par exemple le Sumatriptan ou le Rizatriptan) mais aussi des dérivés de l'ergot pour lesquels de nombreux effets secondaires sont malheureusement relevés : altération du goût ; sécheresse buccale; bouffées de chaleur; endolorissement musculaire ; essoufflement ; étourdissements ; fatigue ; nausée ou des vomissements ; lourdeur; engourdissement; maux de tête; faiblesse et somnolence.

Des thérapies alternatives existent également et sont essentiellement représentées par les suppléments diététiques comprenant de la vitamine B6 et/ou du magnésium (à raison de 200 à 400 mg/jour) et/ou du manganèse et/ou de la vitamine E. Des compléments alimentaires existent également et sont le plus souvent à base d'huile d'onagre ou de bourrache, de vitamine B et de magnésium.

Le magnésium a été identifié assez rapidement comme étant un élément important dans le traitement du SPM. En effet, il est reconnu qu'un déficit en magnésium est commun à presque toutes les formes de syndrome prémenstruel et qu'un apport en ce minéral est favorable à son traitement. C'est d'ailleurs pourquoi des compositions proposées pour le traitement du SPM et comprenant du magnésium, en particulier du magnésium sous forme de sels, sont connues. En effet, divers sels de magnésium sont utilisés de nos jours dans le traitement du SPM, comme par exemple l'oxyde de magnésium ou le thréonate de magnésium. En ce sens, le document EP1661575 divulgue des compositions comprenant en mélange de l'oxyde de magnésium et du stéarate de magnésium. Plus particulièrement, le document EP1661575 divulgue l'utilisation d'une composition pharmaceutique comprenant du magnésium comme agent actif pour une administration par voie orale à libération pulsée dans la prévention ou le traitement des symptômes associés au syndrome prémenstruel, où la libération pulsée imite le rythme circadien des hormones du cycle chez la femme et où la source de magnésium est un sel pharmaceutiquement acceptable comme l'oxyde de magnésium.

Le document de F. Facchinetti et al. (Oral magnesium successfully relieves premenstrual mood changes, Obstet. & Gynecol., vol. 78, no. 2, 1991, pages 177-181) décrit les effets bénéfiques d'une supplémentation en magnésium sous la forme d'acide pyrrolidone carboxylique sur différents symptômes du syndrome prémenstruel.

Le document de S. Quaranta et al. (Pilot study of the efficacy and safety of a modified-release magnesium 250mg tablet (Sincromag®) for the treatment of premenstrual syndrome, Clinical Drug Investigation, Adis International, Auckland, NZ, vol. 27, no. 1, 2007, pages 51-58) décrit les effets bénéfiques de l'administration de magnésium sous une forme à libération modifiée en corrélation avec le rythme circadien de différentes hormones sur des symptômes du syndrome prémenstruel.

Le document de Uysal Nazan et al. (Timeline (Bioavailability) of magnesium compounds in hours: which magnesium compound works best?, Biological Trace Element Research, Humana Press, Clifton, NJ, US, vol. 187, no. 1, 21, 2018, pages 128-136) étudie la biodisponibilité de différentes sources de magnésium dont l'acétyle taurate de magnésium.

Le document de Maria Kharitonova et al. (Comparative angioprotective effects of magnesium compounds, Journal of Trace Elements in Medecine and Biology, vol. 29, 2015, pages 227-234) étudie les effets de différents composés de magnésium dont le N-acétyltaurate de magnésium.

Le document KR20160008257 divulgue l'efficacité de la taurine pour le traitement des troubles menstruels.

Le document WO2016/205089 divulgue l'utilisation combinée de la taurine et d'une supplémentation en magnésium sous forme d'oxyde de magnésium pour le traitement de l'acné prémenstruel.

Le document de J. Durlach et al. (Mg acetyltaurinate as a photic inhibitor in photosensitive magnesium depletion: a physiological pathway in headache with photophobia treatment, 2013, extrait de l'Internet) décrit l'acétyle taurate de magnésium en tant qu'inhibiteur de l'hypersensibilité photique pour le traitement de la migraine due à l'hypersensibilité photique.

Malheureusement, actuellement, il apparait que les traitements médicamenteux principaux donnent lieu à de nombreux effets secondaires et que les traitements alternatifs sont relativement peu efficaces et par conséquent peu utilisés.

A ce jour, il n'existe donc pas de traitement médicamenteux sans effets secondaires et les traitements alternatifs sont peu convaincants. Il existe donc un réel besoin de mettre au point et de formuler un composé et/ou une composition alternative efficace, sure et minimisant voire éliminant au moins en partie les effets secondaires tels que ceux listés plus haut pour assurer un traitement adéquat et optimisé du syndrome prémenstruel et/ou du trouble dysphorique prémenstruel.

Pour résoudre ce problème, il est prévu suivant l'invention, un composé et une composition pour utilisation dans le traitement du syndrome prémenstruel et/ou du trouble dysphorique prémenstruel, ledit composé étant du N-acétyl-taurinate de magnésium et ladite composition comprenant du N-acétyl-taurinate de magnésium.

Dans le cadre de la présente invention, il a été déterminé que le composé selon l'invention, c'est-à-dire le N-acétyl-taurinate de magnésium, est plus efficace (réduction des symptômes) que les autres composés, en particulier plus efficace que les autres sels de magnésium connus de l'état de la technique (comme l'oxyde de magnésium) pour leur utilisation dans le traitement du syndrome prémenstruel et/ou du trouble dysphorique prémenstruel. Par ailleurs, une composition selon l'invention présente un coût acceptable et est à la fois efficace et sûre pour le traitement du syndrome prémenstruel et/ou du trouble dysphorique prémenstruel.

Notons que le N-acétyl-taurinate de magnésium (C₈H₁₆MgN₂O₈S₂) est connu pour ses propriétés cyto-vasculo protectrices telles que des propriétés anti-aggrégantes plaquettaires et protectrices des thromboses veineuses et artérielles ainsi qu'une action stabilisatrice de la membrane érythrocytaire. En outre, un effet protectif du magnésium N-acétyl-taurinate contre le glaucome en agissant sur la dérégulation vasculaire a été démontré, tout comme les propriétés angio-protectrices du N-acétyl-taurinate de magnésium par son action anti-inflammatoire en restaurant les teneurs en eNOS (endothelial Nitric Oxide Synthase 3). Des études ont également démontré un effet bénéfique du magnésium N-acétyl-taurinate sur la neurotoxicité due à l'hyperexcitabilité des récepteurs glutamatergiques ionotropes. En effet, le magnésium N-acétyl-taurinate a une action intraneuronale non seulement sur le récepteur NMDA mais également sur les deux autres récepteurs ionotropes de l'acide glutamique à savoir les récepteurs AMPA et KA, ceux-ci étant impliqués dans la vitesse de la transmission synaptique. Il a été remarqué qu'un tel composé présente une analogie structurelle avec l'acide glutamique mais également l'acide kaïnique. Ainsi, le N-acétyl-taurinate de magnésium va cibler l'ensemble des récepteurs glutamatergiques NMDAR, AMPAR et KAR entrainant par conséquent l'inhibition des voies de signalisation en aval de ces récepteurs.

De façon tout à fait avantageuse, selon l'invention, ledit N-acétyl-taurinate de magnésium est le N-acétyl-taurinate de magnésium dihydraté comprenant deux molécules d'eau intrinsèques.

Par les termes « deux molécules d'eau intrinsèques », il est entendu, au sens de la présente invention, que les deux molécules d'eau sont inhérentes au N-acétyl-taurinate de magnésium dihydraté, c'est-à-dire qu'elles font partie intégrante du N-acétyl-taurinate de magnésium dihydraté, à la différence de l'eau d'hydratation qui pourrait être absorbée ou adsorbée par ce composé.

Le N-acétyl-taurinate de magnésium dihydraté (C₈H₂₀MgN₂O₁₀S₂), dénommé également magnésium N-acétyl-taurinate dihydraté, est un vecteur magnésique et un analogue magnésique de la taurine qui présente un poids moléculaire de 392,677 g/mol, deux molécules d'eau (H₂O) étant intrinsèques à la molécule de N-acétyl-taurinate de magnésium dihydraté.

En particulier, le N-acétyl-taurinate de magnésium dihydraté est celui qui est produit et commercialisé sous la marque ATAMg^{®} par la société Synapharm Industrial Synthesis.

Comme le N-acétyl-taurinate de magnésium non dihydraté, le N-acétyl-taurinate de magnésium dihydraté comprenant deux molécules d'eau intrinsèques possède différentes caractéristiques telles qu'un dérivé β aminé soufré, un acide sulfonique (non carboxylique), un N-acétylé, ne présente pas le caractère amphotère de la taurine (forme Zwitterion, c'est-à-dire qu'une charge positive et une charge négative se trouvent sur le même résidu) et optimise le caractère taurinergique intracellulaire. La charge électrique sur l'azote de la taurine étant supprimée par l'acétylation, seuls les électrons du cation Mg⁺⁺ sont maintenus chélatés par les deux radicaux sulfoniques de la taurine. Cela conduit à un dérivé éthanamide (acétamide) au caractère lipophile plus marqué que celui de la taurine amphotère, ce qui facilite la pénétration au travers les phospholipides membranaires neuronaux. Les dérivés éthanamides (acétamides) caractérisent les molécules utilisées pour leurs actions nootropes (piracetam-like), anti-convulsivantes et anti-épileptiques (levitracetam-like).

Le poids moléculaire du N-acétyl-taurinate de magnésium dihydraté est de 392,677 g/mol et diffère de celui du N-acétyl-taurinate de magnésium non dihydraté (C₈H₁₆MgN₂O₈S₂) qui est de 356,656 g/mol. Cette forme qui n'est pas dihydratée est notamment décrite dans le document FR2384751 où le N-acétyl-taurinate de magnésium est obtenu par mélange de magnésie, de taurine, d'eau et d'acide acétique avant deux étapes successives de séchage, l'une sous vide à 100°C et l'autre avec un solvant de séchage, de telle sorte à obtenir des cristaux.

Par ailleurs, il a été déterminé que le N-acétyl-taurinate de magnésium sous forme dihydratée comprenant deux molécules d'eau intrinsèques présente une stabilité au cours du temps qui est augmentée par rapport au N-acétyl-taurinate de magnésium non dihydraté, c'est-à-dire par rapport au N-acétyl-taurinate de magnésium anhydre. En d'autres termes, il a été mis en évidence que la forme dihydratée du N-acétyl-taurinate de magnésium conserve mieux ses propriétés au cours du temps en comparaison avec la forme anhydre du N-acétyl-taurinate de magnésium.

Il a en outre été déterminé que les deux molécules d'eau intrinsèques sont responsables de la formation d'un complexe stable par établissement de liaisons entre les deux molécules d'eau intrinsèques et le magnésium présents au sein du composé N-acétyl-taurinate de magnésium dihydraté. Plus particulièrement, le magnésium est lié à la N-acétyl-taurine et à l'eau par l'intermédiaire de liaisons non covalentes de type liaisons de coordination métal-ligand.

Par ailleurs, comme indiqué plus haut, le composé selon l'invention est sûr et bénéfique (au-delà de ses propriétés exposées plus haut) pour le corps humain. En effet, le magnésium contribue au maintien de la balance des fluides et des électrolytes, à un métabolisme énergétique et une synthèse protéique normaux, à des fonctions musculaires adaptées, à la réduction de la fatigue et de l'asthénie, à maintenir une bonne dentition et une structure osseuse adéquate et contribue au fonctionnement normal du système nerveux et de la fonction psychique.

En outre, le N-acétyl-taurinate aide, quant à lui, la pénétration cellulaire du magnésium et de la taurine et agit sur le maintien de l'osmolarité cellulaire.

De préférence, le composé se présente sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

Dans une forme particulière selon l'invention, le composé est administré à raison de 2 à 3 capsules par jour.

D'autres formes de réalisation du composé suivant l'invention sont indiquées dans les revendications annexées.

La présente invention porte également sur une composition pour utilisation dans le traitement du syndrome prémenstruel et/ou du trouble dysphorique prémenstruel.

De façon tout à fait avantageuse, dans une composition selon l'invention, ledit N-acétyl-taurinate de magnésium est le N-acétyl-taurinate de magnésium dihydraté comprenant deux molécules d'eau intrinsèques.

Par les termes « deux molécules d'eau intrinsèques », il est entendu, au sens de la présente invention, que les deux molécules d'eau sont inhérentes au N-acétyl-taurinate de magnésium dihydraté, c'est-à-dire qu'elles font partie intégrante du N-acétyl-taurinate de magnésium dihydraté, à la différence de l'eau d'hydratation qui pourrait être absorbée ou adsorbée par ce composé.

Le N-acétyl-taurinate de magnésium dihydraté (C₈H₂₀MgN₂O₁₀S₂), dénommé également magnésium N-acétyl-taurinate dihydraté, est un vecteur magnésique et un analogue magnésique de la taurine qui présente un poids moléculaire de 392,677 g/mol, deux molécules d'eau (H₂O) étant intrinsèques à la molécule de N-acétyl-taurinate de magnésium dihydraté.

En particulier, le N-acétyl-taurinate de magnésium dihydraté est celui qui est produit et commercialisé sous la marque ATAMg^{®} par la société Synapharm Industrial Synthesis.

Le poids moléculaire du N-acétyl-taurinate de magnésium dihydraté est de 392,677 g/mol et diffère de celui du N-acétyl-taurinate de magnésium non dihydraté (C₈H₁₆MgN₂O₈S₂) qui est de 356,656 g/mol. Cette forme qui n'est pas dihydratée est notamment décrite dans le document FR2384751 où le N-acétyl-taurinate de magnésium est obtenu par mélange de magnésie, de taurine, d'eau et d'acide acétique avant deux étapes successives de séchage, l'une sous vide à 100°C et l'autre avec un solvant de séchage, de telle sorte à obtenir des cristaux.

Il a en outre été déterminé que les deux molécules d'eau intrinsèques sont responsables de la formation d'un complexe stable par établissement de liaisons entre les deux molécules d'eau intrinsèques et le magnésium présents au sein du composé N-acétyl-taurinate de magnésium dihydraté. Plus particulièrement, le magnésium est lié à la N-acétyl-taurine et à l'eau par l'intermédiaire de liaisons non covalentes de type liaisons de coordination métal-ligand.

La formation d'un tel complexe est particulièrement avantageuse puisque ce dernier assure une stabilité accrue du composé : le magnésium reste fixé (piégé) au complexe.

Avantageusement, le N-acétyl-taurinate de magnésium ou le N-acétyl-taurinate de magnésium dihydraté comprenant deux molécules d'eau intrinsèques est présent à raison de 40 à 60% en poids par rapport au poids total de la composition.

Dans une forme particulièrement avantageuse, la composition selon l'invention comprend en outre de la taurine à raison de 10 à 15% en poids par rapport au poids total de la composition.

Avantageusement, la composition selon l'invention comprend en outre au moins un composé magnésique additionnel choisi dans le groupe constitué des sels magnésiques d'acides aminés, du citrate de magnésium, du gluconate de magnésium, du lactate de magnésium, du malate de magnésium, du taurate de magnésium, du bisglycinate de magnésium, du glycérophosphate de magnésium, du thréonate de magnésium, du pidolate de magnésium, leurs dérivés et leurs mélanges.

De préférence, selon l'invention, la composition comprend en outre du magnésium à raison de 8 à 15% en poids par rapport au poids total de la composition.

De plus, dans une forme de réalisation particulière, la composition comprend en outre au moins un acide aminé, par exemple de la thréonine.

De préférence, ledit au moins un acide aminé est la glycine présente à raison de 10 à 15% en poids par rapport au poids total de la composition.

De manière avantageuse, ledit au moins un acide aminé est la thréonine présente à raison de 10 à 15% en poids par rapport au poids total de la composition.

Dans une forme particulière, selon l'invention, la composition comprend en outre au moins une vitamine choisie dans le groupe constitué de la vitamine B1, B2, B6 et leurs mélanges.

Avantageusement, selon l'invention, ladite au moins une vitamine est la vitamine B1 présente à raison de 0,05 à 0,1% en poids par rapport au poids total de la composition.

De préférence, ladite au moins une vitamine est la vitamine B2 présente à raison de 0,05 à 0,1% en poids par rapport au poids total de la composition.

Dans une forme de réalisation particulièrement avantageuse, selon l'invention, ladite au moins une vitamine est la vitamine B6 présente à raison de 0,05 à 0,15 % en poids par rapport au poids total de la composition.

De façon avantageuse, la composition comprend en outre au moins un excipient pharmaceutiquement acceptable, de préférence quatre excipients pharmaceutiquement acceptables, choisi dans le groupe constitué du glycéryl béhénate, du stéarate de saccharose, de la cellulose microcristalline, du stéarate de magnésium, du dioxyde de silice, de la malodextrine de pois et leurs mélanges.

Dans une forme particulière, la composition selon l'invention se présente sous la forme d'un médicament.

De préférence, la composition selon l'invention se présente sous la forme d'un complément alimentaire.

Avantageusement, la composition selon l'invention se présente sous la forme d'une boisson.

Dans une forme de réalisation particulièrement avantageuse selon l'invention, la composition se présente sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

De plus, dans une forme de réalisation particulière, la composition selon l'invention est administrée à raison de 2 à 3 capsules par jour.

D'autres formes de réalisation de la composition suivant l'invention sont indiquées dans les revendications annexées.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composé pour utilisation dans le traitement du syndrome prémenstruel et/ou du trouble dysphorique prémenstruel, ledit composé étant du N-acétyl-taurinate de magnésium.

2. Composé pour utilisation selon la revendication 1, ledit N-acétyl-taurinate de magnésium étant du N-acétyl-taurinate de magnésium dihydraté comprenant deux molécules d'eau intrinsèques.

3. Composé pour utilisation selon la revendication 1 ou 2, ledit composé se présentant sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

4. Composition pour utilisation dans le traitement du syndrome prémenstruel et/ou du trouble dysphorique prémenstruel, ladite composition comprenant du N-acétyl-taurinate de magnésium.

5. Composition pour utilisation selon la revendication 4, **caractérisée en ce que** ledit N-acétyl-taurinate de magnésium est du N-acétyl-taurinate de magnésium dihydraté comprenant deux molécules d'eau intrinsèques.

6. Composition pour utilisation selon la revendication 4 ou 5, dans laquelle ledit N-acétyl-taurinate de magnésium ou ledit N-acétyl-taurinate de magnésium dihydraté comprenant deux molécules d'eau intrinsèques est présent à raison de 40 à 60% en poids par rapport au poids total de la composition.

7. Composition pour utilisation selon l'une quelconque des revendications 4 à 6, laquelle comprend en outre de la taurine à raison de 10 à 15% en poids par rapport au poids total de la composition.

8. Composition pour utilisation selon l'une quelconque des revendications 4 à 7, laquelle comprend en outre au moins un composé magnésique additionnel choisi dans le groupe constitué des sels magnésiques d'acides aminés, du citrate de magnésium, du gluconate de magnésium, du lactate de magnésium, du malate de magnésium, du taurate de magnésium, du bisglycinate de magnésium, du glycérophosphate de magnésium, du thréonate de magnésium, du pidolate de magnésium, et leurs mélanges.

9. Composition pour utilisation selon l'une quelconque des revendications 4 à 8, comprenant en outre au moins un excipient pharmaceutiquement acceptable, de préférence quatre excipients pharmaceutiquement acceptables, choisi dans le groupe constitué du glycéryl béhénate, du stéarate de saccharose, de la cellulose microcristalline, du stéarate de magnésium, du dioxyde de silice, de la malodextrine de pois et leurs mélanges.

10. Composition pour utilisation selon l'une quelconque des revendications 4 à 9, ladite composition se présentant sous la forme d'un médicament ou d'un complément alimentaire ou d'une boisson.

11. Composition pour utilisation selon l'une quelconque des revendications 4 à 10, ladite composition se présentant sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

## Patentansprüche

1. Verbindung zur Verwendung bei der Behandlung des prämenstruellen Syndroms und/oder prämenstrueller dysphorischer Störungen, wobei die genannte Verbindung N-Acetyl-Magnesiumtaurinat ist.

2. Verbindung zur Verwendung gemäß Anspruch 1, wobei das genannte N-Acetyl-Magnesiumtaurinat N-Acetyl-Magnesiumtaurinatdihydrat, umfassend zwei intrinsische Wassermoleküle, ist.

3. Verbindung zur Verwendung gemäß Anspruch 1 oder 2, wobei die genannte Verbindung eine oral verabreichbare Form, zum Beispiel die Form einer Tablette, einer Kapsel, einer Gelatinekapsel, einer Pille, eines löslichen Pulvers, einer ölhaltigen Lösung, einer Brausetablette oder einer weichen Gelatinekapsel aufweist.

4. Zusammensetzung zur Verwendung bei der Behandlung des prämenstruellen Syndroms und/oder prämenstrueller dysphorischer Störungen, wobei die genannte Zusammensetzung N-Acetyl-Magnesiumtaurinat umfasst.

5. Zusammensetzung zur Verwendung gemäß Anspruch 4, **dadurch gekennzeichnet, dass** das genannte N-Acetyl-Magnesiumtaurinat N-Acetyl-Magnesiumtaurinatdihydrat ist, umfassend zwei intrinsische Wassermoleküle.

6. Zusammensetzung zur Verwendung gemäß Anspruch 4 oder 5, bei der das genannte N-Acetyl-Magnesiumtaurinat oder das genannte N-Acetyl-Magnesiumtaurinatdihydrat, zdas wei intrinsische Wassermoleküle umfasst, im Umfang von 40 bis 60 Gew.-% in Bezug auf das Gesamtgewicht der Zusammensetzung vorhanden ist.

7. Zusammensetzung zur Verwendung gemäß irgendeinem der Ansprüche 4 bis 6, die darüber hinaus das Taurin im Umfang von 10 bis 15 Gew.-% in Bezug auf das Gesamtgewicht der Zusammensetzung umfasst.

8. Zusammensetzung zur Verwendung gemäß irgendeinem der Ansprüche 4 bis 7, die darüber hinaus wenigstens eine zusätzliche Magnesiumverbindung umfasst, die aus der Gruppe ausgewählt ist, welche aus Magnesiumsalzen von Aminosäuren, Magnesiumcitrat, Magnesiumglukomat, Magnesiumlaktat, Magnesiummalat, Magnesiumtaurat, Magnesiumbisglycinat, Magnesiumglycerophosphat, Magnesiumthreonat, Magnesiumpidolat und deren Mischungen gebildet ist.

9. Zusammensetzung zur Verwendung gemäß irgendeinem der Ansprüche 4 bis 8, umfassend darüber hinaus wenigstens einen pharmazeutisch akzeptablen Hilfsstoff, bevorzugt vier pharmazeutisch akzeptable Hilfsstoffe, die aus der Gruppe ausgewählt sind, die aus Glycerylbehenat, Saccharosestearat, mikrokristalliner Zellulose, Magnesiumstearat, Siliziumdioxid, Erbsenmalodextrin und deren Mischungen gebildet ist.

10. Zusammensetzung zur Verwendung gemäß irgendeinem der Ansprüche 4 bis 9, wobei die genannte Zusammensetzung die Form eines Medikaments oder einer Nahrungsmittelergänzung oder eines Getränks aufweist.

11. Zusammensetzung zur Verwendung gemäß irgendeinem der Ansprüche 4 bis 10, wobei die genannte Zusammensetzung eine oral verabreichbare Form, zum Beispiel die Form einer Tablette, einer Kapsel, einer Gelatinekapsel, einer Pille, eines löslichen Pulvers, einer ölhaltigen Lösung, einer Brausetablette oder einer weichen Gelatinekapsel aufweist.

## Claims

1. A compound for use in the treatment of premenstrual syndrome and/or premenstrual dysphoric disorder, said compound being magnesium N-acetyl-taurinate.

2. The compound for use according to claim 1, said magnesium N-acetyl-taurinate being magnesium N-acetyl-taurinate dihydrate comprising two intrinsic water molecules.

3. The compound for use according to claim 1 or 2, said compound being in an orally administrable form, for example in the form of a tablet, capsule, hard capsule, cachet, soluble powder, oily solution, effervescent tablet or soft capsule.

4. A composition for use in the treatment of premenstrual syndrome and/or premenstrual dysphoric disorder, said composition comprising magnesium N-acetyl-taurinate.

5. The composition for use according to claim 4, **characterized in that** said magnesium N-acetyl-taurinate is magnesium N-acetyl-taurinate dihydrate comprising two intrinsic water molecules.

6. The composition for use according to claim 4 or 5, wherein said magnesium N-acetyl-taurinate or said magnesium N-acetyl-taurinate dihydrate comprising two intrinsic water molecules is present in an amount between 40 and 60% by weight based on the total weight of the composition.

7. The composition for use according to any one of claims 4 to 6, which further comprises taurine in an amount between 10 and 15% by weight based on the total weight of the composition.

8. The composition for use according to any one of claims 4 to 7, which further comprises at least one additional magnesium compound selected from the group consisting of magnesium salts of amino acids, magnesium citrate, magnesium gluconate, magnesium lactate, magnesium malate, magnesium taurate, magnesium bisglycinate, magnesium glycerophosphate, magnesium threonate, magnesium pidolate, and mixtures thereof.

9. The composition for use according to any one of claims 4 to 8, further comprising at least one pharmaceutically acceptable excipient, preferably four pharmaceutically acceptable excipients, selected from the group consisting of glyceryl behenate, sucrose stearate, microcrystalline cellulose, magnesium stearate, silica dioxide, pea malodextrin and mixtures thereof.

10. The composition for use according to any one of claims 4 to 9, said composition being in the form of a medicament or food supplement or drink.

11. The composition for use according to any one of claims 4 to 10, said composition being in an orally administrable form, for example in the form of a tablet, capsule, hard capsule, cachet, soluble powder, oily solution, effervescent tablet or soft capsule.
